# FASCICULE DE BREVET EUROPEEN

(11) **EP 0 710 477 B1**
(45) Date de publication et mention de la délivrance du brevet: **04.10.2001**
(21) Numéro de dépôt: 94117482.3
(22) Date de dépôt: 05.11.1994
(51) Int. Cl.: A61K 7/48, A23D 9/007

(54) **Composition lipidique pour produits cosmétiques**
Lipidzusammensetzung für Kosmetik
Lipid composition for cosmetic products

(43) Date de publication de la demande: 08.05.1996
(73) Titulaire: SOCIETE DES PRODUITS NESTLE S.A., 1800 Vevey (CH)
(72) Inventeur: Bertoli, Constantin, CH-1032 Romanel S/Lausanne (CH); Bracco, Umberto, CH-1800 Vevey (CH); Delvecchio, Angiolino, CH-6807 Taverne TI (CH); Malnoe, Armand, CH-1261 Givrins (CH)
(74) Mandataire: Archambault, Jean

(56) Documents cités:
- CA-A- 2 088 529
- FR-A- 2 692 783
- US-A- 3 988 436
- US-A- 4 810 498
- Database WPI, AN-1987-047625 & JP-A-62 006661, *abrégé*
- Database WPI, AN-1985-273621 & JP-A-60 186253, *abrégé*
- Database WPI, AN-1992-164263 & JP-A-4104762, *abrégé*
- Lexikon der Hilfsstoffe für Pharmazie, Kosmetik und angrenzende Gebiete, P. Fiedler, Editio Cantor Aulendorf, 1989, p. 667 - 668; 771, 1068; 1098
- Merck Index, 9th ed., 1976, p. 1067
- DATABASE WPI, semaine 1907, Derwent Publications Ltd., Londres, GB, Classe , AN & JP 62006661 A
- DATABASE WPI, semaine 1944, Derwent Publications Ltd., Londres, GB, Classe , AN & JP 60186253 A
- DATABASE WPI, semaine 1920, Derwent Publications Ltd., Londres, GB, Classe , AN & JP 4104762 A

## Description

La présente invention concerne une composition lipidique destinée à être utilisée dans des compositions cosmétiques, notamment une composition lipidique ayant une action anti-vieillissement.

Le vieillissement prématuré de l'épiderme est dû en partie aux agressions extérieures telles que par exemple le rayonnement UV et la pollution générateurs de radicaux libres.

On sait, par exemple de EP-A-0477825, que l'huile de sésame a la propriété de stabiliser les huiles végétales comestibles riches en acides gras insaturés, par exemple l'huile de maïs, contre l'oxydation.

On connaît également, par exemple de EP-A-0581624, les propriétés anti-radicalaires des fractions insaponifiables d'huile de sésame et d'huile de germes de blé dans les compositions cosmétiques.

La présente invention a pour but de proposer une composition lipidique pour produits cosmétiques ayant une action anti-vieillissement de la peau, c'est à dire anti-radicalaire, calmante et hydratante, tout en étant naturellement stabilisée contre l'oxydation, c'est à dire ne contenant pas d'antioxydants ajoutés.

La composition lipidique selon l'invention est caractérisée par le fait qu'elle contient de l'huile de son de riz et de l'huile de sésame, 2 à 3 % en poids d'insaponifiables et que les acides gras des triglycérides comprennent, en poids,
30 % à 40 % d'acide oléique, 40 à 50 % d'acide linoléique et moins de 2 % d'acide alpha-linolénique.

La composition contient de l'huile de son de riz qui est particulièrement riche en gamma-oryzanol ayant une activité antioxydante.

La composition contient également de l'huile de sésame dont certains constituants inhibent spécifiquement la delta 5 désaturase, l'enzyme responsable de la biotransformation de l'acide dihomogamma-linolénique (DHGLA) en acide arachidonique (AA). Elle est donc susceptible d'avoir une action anti-inflammatoire dans la mesure où la formation des produits dérivés de l'AA comme, par exemple, le leucotriène B4, qui est pro-inflammatoire devrait diminuer au profit des produits dérivés du DHGLA, par exemple les prostaglandines de série 1 ayant une activité anti-inflammatoire.

La composition contient une huile comprenant une quantité appréciable d'acide oléique qui a une action de structure et de véhicule des acides gras bio-actifs essentiels tout en étant neutre du point de vue de la bioactivité. La teneur en acide oléique confère au mélange lipidique une bonne stabilité à l'oxydation et à la photo-oxydation, ce qui évite la formation de radicaux oxygénés actifs.

Les huiles de choix répondant à ces exigences sont de préférence, l'huile de son de riz et l'huile de sésame. Les huiles en question constituent de préférence 45 à 65 %, par exemple environ 60 % en poids du mélange lipidique final.

La composition contient des huiles apportant les acides gras essentiels de la famille n-6 et pauvres en acides gras n-3, pour tenir compte de la plus grande réactivité biochimique de ceux de la famille n-3.

Les huiles riches en acide gras de la famille n-6 sont choisies parmi celles riches en acide linoléique, par exemple l'huile de maïs, l'huile de germes de blé, l'huile de tournesol ou l'huile de pépins de raisin. Ainsi, la composition a une teneur élevée en acide linoléique, de 40 à 50 % en poids des acides gras. Cet acide est un constituant des céramides qui jouent le rôle important de barrière contre la deshydratation de l'épiderme. L'acide linoléique se révèle également actif contre l'hyperprolifération cellulaire associée à la carence en acides gras essentiels, un effet qui pourrait être lié au rétablissement de taux normaux de prostaglandines de série 2 dans l'épiderme.

Ces acides gras "actifs" peuvent aussi être incorporés à la formulation sous forme de leurs esters éthyliques ou propioniques, en quantités calculées de façon à obtenir les taux et les proportions relatives souhaitées.

La composition selon l'invention contient encore une huile riche en vitamine E susceptible d'améliorer ses propriétés de conservation, par exemple une huile de germes de blé.

La composition moyenne des acides gras des triglycérides de la composition finale est la suivante:

| **Acides gras** | **% en poids** | | **% en poids** |
|---|---|---|---|
| C16:0 | 10-15 | de préférence | <13,5 |
| C16:1, n-7 | 0,05-0,5 | " | <0,3 |
| C18:0 | 1-4 | " | <3 |
| C18:1, n-9 | 30-50 | " | <35 |
| C18:2, n-6 | 40-60 | " | <48 |
| C18:3, n-3 (alpha) | 1-2 | " | <2 |
| C20:0 | <1 | " | <0,5 |
| C20:1 | **<**1 | " | <0,5 |

Sur la base de leur compositions respectives en acides gras et en constituants antioxydants naturels, les mélanges des huiles ci-après sont préférés:

| **Huile** | **% en poids** | | **% en poids** |
|---|---|---|---|
| Huile de son de riz | 30-40 | de préférence | 40 |
| Huile de maïs | 20-40 | " | 30 |
| Huile de sésame | 15-25 | " | 20 |
| Huile de germes de blé | 5-15 | " | 10 |

L'invention concerne également un procédé de préparation d'une composition lipidique précédente, dans lequel on met en oeuvre des huiles brutes ou partiellement raffinées, caractérisé par le fait que l'on dégomme le mélange d'huiles, on le décolore et on le désodorise dans des conditions permettant de maintenir une teneur en insaponifiables d'au moins 2 % en poids ainsi qu'une stabilité contre l'oxydation correspondant à une période d'induction d'au moins 15 h dans le test Rancimat à 100° C.

Selon l'invention, on met en oeuvre préférablement l'huile de sésame pressée à froid, qui contient les composés souhaités sésamoline et sésamine. Les huiles de germes de blé et de sésame sont de préférence choisies pauvres en lécithines et riches en insaponifiables.

Le dégommage a lieu, de préférence, par mise en contact du mélange d'huiles avec une solution concentrée d'acide citrique, en présence d'eau à environ 80° C, au cours duquel on réalise une hydratation des gommes, puis séparation des gommes, par exemple par centrifugation ou décantation.

Dans une variante du traitement de dégommage, on traite le mélange d'huiles chauffé à environ 80° C par circulation de vapeur sous vide.

Après séparation des gommes comme indiqué précédemment, on traite le mélange d'huiles dégommé par mise en contact avec un adsorbant constitué de gel de silice amorphe humide pendant environ 20 min à environ 80-85° C sous un vide d'environ 50-80 mbar.

Dans certains cas, on procède également à un blanchiment par terre décolorante activée à l'acide.

Enfin, on réalise une désodorisation du mélange d'huiles dans des conditions ménagées, par exemple à environ 180° C avec environ 1 % de vapeur vive et sous un vide d'environ 1-2 mbar pendant environ 2 h. On peut ainsi maintenir une teneur appréciable en insaponifiables et en particulier ménager les tocophérols.

La composition lipidique selon l'invention peut être avantageusement utilisée dans diverses compositions cosmétiques aqueuses ou anhydres pour le traitement de la peau, notamment dans des compositions aqueuses comme les fluides, crèmes et laits pour le visage, les mains et le corps, les crèmes et laits anti-solaires, les crèmes et les laits antirides et les compositions similaires.

La composition cosmétique en question peut se présenter notamment sous la forme de solution, d'émulsion eau-dans-l'huile ou huile-dans-l'eau, de suspension ou d'aérosol. Comme compositions cosmétiques anhydres incorporant la composition lipidique selon l'invention, on peut citer les huiles pour le corps, les baumes anhydres, les huiles anti-solaires et les rouges à lèvres.

Dans une telle composition cosmétique, la composition lipidique selon l'invention peut représenter 1 à 80%, et de préférence 5 à 60 % en poids.

Une telle composition cosmétique renferme généralement, en quantités appropriées, des adjuvants tels que, par exemple les émulsifiants, les agents anti-transpirants, les stabilisants, les conservateurs, les filtres solaires, les parfums, les colorants ou les émollients, les cires, les agents nacrants, les charges minérales ou organiques.

La composition lipidique selon l'invention peut également être utilisée dans un but cosmétique sous forme de supplément nutritionnel, par exemple en capsules ou gélules.

Les exemples ci-après illustrent l'invention. Dans ceux-ci, les pourcentages et parties sont pondéraux sauf indication contraire.

### Exemples 1-3

### Préparation du mélange d'huiles

On mélange sous agitation et sous azote les huiles partiellement raffinées ci-après dans les proportions indiquées.

| **Huile** | **%** |
|---|---|
| Huile de son de riz | 40 |
| Huile de maïs | 30 |
| Huile de sésame | 20 |
| Huile de germe de blé | 10 |

Pour ce faire, dans un réacteur en acier inoxydable muni d'un système de double manteau à circulation de fluides pour thermostatisation et d'un agitateur à vitesse variable, on mélange les huiles dans les proportions indiquées ci-dessus en évitant des températures supérieures à 30°C.

**Exemple 1:** On chauffe ensuite le mélange à 65° C et on le traite avec 0,3 % d'acide citrique à 50 %, puis on ajoute 2 à 3 % d'eau et on sépare par centrifugation les gommes précipitées.

On met ensuite le mélange dégommé en contact avec 1 % de silicagel amorphe hydraté (TriSyl (R)) et 0,5 % silicagel amorphe hydraté (TriSyl 300(R)) à 80-85° C pendant 20 min sous un vide de 50-80 mbar.

On désodorise enfin le mélange à 180° C pendant 3 h par entraînement à la vapeur avec 1 % de vapeur par h.

Les propriétés du mélange d'huiles raffiné final sont les suivantes:

| | |
|---|---|
| Couleur Lovibond (R), cellule de 2,5 cm (1"), R | 0,9 |
| Couleur Lovibond (R), cellule de 2,5 cm (1"), R | 5,3 |
| Temps d'induction, test Rancimat (R), h | 17,5 |
| Teneur en insaponifiables, mesuré par la méthode IUPAC 2.104, g/kg | 21,3 |
| Teneur en acides gras libres, % | 0,28 |

**Exemple 2**: On procède comme à l'exemple 1 précédent, mis à part le fait que l'on effectue le dégommage préliminaire par traitement à la vapeur à 80° C pendant 20 min avec 2 % de vapeur. Le résultat des analyses de couleur Lovibond et du test Rancimat sont identiques.

**Exemple 3:** On procède comme à l'exemple 2, à celà près que l'on met le mélange dégommé en contact avec 0,5 % de TriSyl 300(R) et que l'on fait suivre ce traitement par une mise en contact avec 0,25 % de terre décolorante Tonsil Optimum FF(R) avant la désodorisation. Les résultats de coloration et de temps d'induction sont les suivants:
Couleur Lovibond (R), cellule de 2,5 cm 1,4
   (1"), R
Couleur Lovibond (R), cellule de 2,5 cm 9,5
   (1"),Y
Temps d'induction, test Rancimat (R), h 16,5

### Exemple 4

| **Baume anhydre** | |
|---|---|
| **Ingrédient** | **%** |
| Lanoline | 35 |
| Lanoline hydrogénée | 30 |
| Ozokérite | 3 |
| Composition lipidique selon l'exemple 2 | 20 |
| Cyclopentadiméthylsiloxane | 12 |

On obtient le produit anhydre précédent par mélange des constituants à 70° C, puis refroidissement sous brassage, jusqu'à la température ambiante.

### Exemple 5

| **Rouge à lèvres (anhydre) Ingrédients** | **%** |
|---|---|
| Esters d'alcools gras C8-C10 | 26 |
| Ozokérite | 10 |
| Cire de carnauba | 3 |
| Cire d'abeille | 3 |
| Pigment | 9 |
| Parfum | 0,1 |
| Huile de ricin | qsp 100 |
| Composition lipidique de l'exemple 2 | 6 |

On tamise les pigments. On mélange ensuite les constituants à 70° C, sauf le parfum. On laisse refroidir sous brassage jusqu'à 35° C, puis on ajoute le parfum. On passe enfin la préparation au laminoir à trois cylindres.

### Exemple 6

| **Fond de teint Ingrédients** | **%** |
|---|---|
| Composition lipidique de l'exemple 2 | 4 |
| Mélange de mono-di-stéarate de glycéryle, acide stéarique, glycérine (40/50/5/5) | 3,3 |
| Mélange d'alcool de lanoline, huile de vaseline(15/85) | 3 |
| Mono-di-iso-stéarate de glycéryle | 1,8 |
| Palmitate d'isopropyle | 5 |
| Palmitate d'éthyl-2-hexyle | 5 |
| Oxyde de titane | 8,31 |
| Oxyde de fer brun | 0,73 |
| Oxyde de fer jaune | 1,7 |
| Oxyde de fer noir | 0,26 |
| p-Hydroxybenzoate de propyle | 0,1 |
| p-Hydroxybenzoate de méthyle | 0,1 |
| Parfum | 0,3 |
| Triéthanolamine | 1,2 |
| Silicate de magnésium et d'aluminium hydrate | 1,5 |
| Carboxyméthylcellulose de sodium | 0,14 |
| Cyclopentadiméthylsiloxane | 8 |
| Glycérine | 3 |
| Eau déminéralisée stérilisée | qsp100 |
| Propylène glycol | 3 |
| Acide stéarique | 2,4 |

On mélange les pigments et on les tamise, puis on les incorpore dans la phase huileuse, préalablement chauffée à 70° C. A part, on disperse le carboxyméthylcellulose de sodium dans l'eau. Lorsque la solution est homogène, on ajoute les autres composants de la phase aqueuse et on chauffe celle-ci à 75° C. On émulsifie ensuite les deux phases avec homogénéisation rapide. On laisse refroidir sous brassage, on ajoute le parfum et la triéthanolamine à 35° C, puis on homogénéise. On passe enfin la préparation au laminoir à trois cylindres.

### Exemple 7

| **Lait corporel protecteur hydratant** **Ingrédients** | **%** |
|---|---|
| Polysorbate 60 | 0,8 |
| Parfum | 0,3 |
| Glycérol stéarate et PEG 100 stéarate | 1 |
| Polyisobutène hydrogéné | 2 |
| Composition lipidique de l'exemple 2 | 8 |
| Acide stéarique | 1 |
| Glycérine | 3 |
| Carbopol 941 | 0,3 |
| Triéthanolamine | 0,3 |
| Eau + conservateur | qsp 100 |

On disperse le Carbopol 941 dans l'eau. Lorsque la solution est homogène, on ajoute les autres composants de la phase aqueuse et on chauffe celle-ci à 75° C. A part, on mélange les constituants de la phase huileuse à 70° C. On procède ensuite à l'émulsification des deux phases sous homogénéisation rapide. On laisse refroidir sous brassage et on ajoute le parfum, la triéthanolamine et le conservateur à 35° C, puis on homogénéise. On laisse refroidir à la température ambiante et on conditionne.

### Exemple 8

| **Fluide de soin protecteur Ingrédients** | **%** |
|---|---|
| Méthyl glucose sesquistéarate | 2 |
| Composition lipidique de l'exemple 2 | 2 |
| Cyclométhicone | 13 |
| Parfum | 0,2 |
| PEG 20 méthyl glucose sesquistéarate | 2 |
| Gomme de xanthane | 0,2 |
| Polyacrylamide acide et C13-C14-isoparaffine et laureth 7 | 0,8 |
| Eau + conservateurs | qsp 100 |

On disperse la gomme de xanthane dans l'eau à 75° C. A part, on mélange les constituants de la phase huileuse à 70° C. On émulsifie alors les deux phases sous homogénéisation rapide. On laisse ensuite refroidir sous brassage, on ajoute le parfum et le conservateur à 35° C, puis on homogénéise. On laisse enfin refroidir pusqu'à la température ambiante et on conditionne.

### Exemple 9

| **Crème de soin protectrice, émulsion huile-dans-l'eau Ingrédients** | **%** |
|---|---|
| PEG 20 stéarate | 1 |
| Glycéryl stéarate et PEG 100 stéarate | 1 |
| Acide stéarique | 1 |
| Alcool stéarylique | 2 |
| Composition lipidique de l'exemple 2 | 20 |
| Hydrolysat de protéine de soja | 0,2 |
| Glycérine | 3 |
| Carbopol 941 | 0,4 |
| Triéthanolamine | 0,4 |
| Eau + conservateur | qsp 100 |

On disperse le Carbopol 941 dans l'eau. Lorsque la solution est homogène, on ajoute les autres composants de la phase aqueuse et on chauffe celle-ci à 70° C. A part, on mélange les constituants de la phase huileuse à 75° C. On procède ensuite à l'émulsification des deux phases sous homogénéisation rapide. On laisse refroidir sous brassage et on ajoute le parfum à 35° C, puis on homogénéise. On laisse refroidir à la température ambiante et on conditionne.

### Exemple 10

| **Crème de soins, émulsion eau-dans-l'huile** **Ingrédients** | **%** |
|---|---|
| Monoisostéarate de sorbitane | 5 |
| Cire microcristalline | 1 |
| Composition lipidique de | 19 |
| l'exemple 2 | |
| Esters d'acides gras en C8-C10 et d'alcools gras en C12-C18 | 1 |
| Gel de montmorillonite modifié et d'huile neutre (triglycérides d'acides caprylique et caprique) | 5 |
| Propylène glycol | 3 |
| Eau + conservateur | qsp 100 |

On mélange les constituants de la phase huileuse à 75° C. A part, on chauffe les constituants de la phase aqueuse à 70° C. Après émulsification des deux phases sous homogénéisation rapide, on laisse refroidir sous brassage jusqu'à la température ambiante et on conditionne.

## Revendications

1. Composition lipidique pour usage cosmétique, ayant une action anti-vieillissement, **caractérisée par** le fait qu'elle contient de l'huile de son de riz et de l'huile de sésame, 2 à 3 % en poids d'insaponifiables et que les acides gras des triglycérides comprennent, en poids, 30 % à 40 % d'acide oléique, 40 à 50 % d'acide linoléique et moins de 2 % d'acide alpha-linolénique.

2. Composition lipidique selon la revendication 1, **caractérisée par** le fait qu'elle contient, en poids, 30 à 50 % d'huile de son de riz, 15 à 25 % d'huile de sésame, 20 à 40 % d'huile de mais et 5 à 15 % d'huile de germe de blé.

3. Procédé de préparation d'une composition lipidique selon la revendication 1, dans lequel on met en oeuvre des huiles brutes ou partiellement raffinées, **caractérisé par** le fait que l'on dégomme le mélange d'huiles, on le décolore et on le désodorise dans des conditions permettant de maintenir une teneur en insaponifiables d'au moins 2 % en poids ainsi qu'une stabilité contre l'oxydation correspondant à une période d'induction d'au moins 15 h dans le test Rancimat à 100° C.

4. Procédé selon la revendication 3, **caractérisé par** le fait que le dégommage a lieu, par mise en contact du mélange d'huiles avec une solution concentrée d'acide citrique, en présence d'eau à environ 80° C, au cours duquel on réalise une hydratation des gommes, puis séparation des gommes, par centrifugation ou décantation.

5. Procédé selon la revendication 3, **caractérisé par** le fait que le dégommage a lieu, par traitement du mélange d'huiles chauffé à environ 80° C par circulation de vapeur sous vide.

6. Procédé selon la revendication 3, **caractérisé par** le fait que, après séparation des gommes, on traite le mélange d'huiles dégommé par mise en contact avec un adsorbant constitué de gel de silice amorphe humide pendant environ 20 min à environ 80-85° C sous un vide d'environ 50-80 mbar.

7. Procédé selon la revendication 3, **caractérisé par** le fait que l'on procède également à un blanchiment par terre décolorante activée à l'acide.

8. Procédé selon la revendication 3, **caractérisé par** le fait que l'on désodorise le mélange d'huiles dans des conditions ménagées, à environ 180° C avec environ 1 % de vapeur vive et sous un vide d'environ 1-2 mbar pendant environ 2 h.

9. Composition cosmétique dont la partie lipidique est constituée d'une composition lipidique selon la revendication 1 ou 2.

10. Composition cosmétique selon la revendication 9, **caractérisée par** le fait qu'elle se présente sous forme aqueuse, de solution, de suspension ou d'aérosol, d'émulsion eau-dans-l'huile, notamment de crème ou huile-dans-l'eau, notamment de lait ou sous forme anhydre, notamment de baume, d'huile pour le corps, d'huile anti-solaire ou de rouge à lèvres et qu'elle contient également au moins un adjuvant cosmétique choisi dans le groupe formé par les émulsifiants, les agents anti-transpirants, les stabilisants, les conservateurs, les antioxydants, les filtres solaires, les parfums, les colorants, les émollients, les agents nacrants, les cires et les charges organiques ou minérales.

11. Supplément nutritionnel à but cosmétique dont la partie lipidique est constituée d'une composition lipidique selon la revendication 1 ou 2.

## Patentansprüche

1. Lipidzusammensetzung zu kosmetischen Zwecken mit einer Wirkung gegen Altern, **dadurch gekennzeichnet, dass** sie Reiskleieöl und Sesamöl und 2 bis 3 Gew.-% Unverseifbares enthält und **dass** die Fettsäuren der Triglyceride, bezogen auf das Gewicht, 30 bis 40% Ölsäure, 40 bis 50% Linolsäure und weniger als 2% α-Linolensäure umfassen.

2. Lipidzusammensetzung nach Anspruch 1, **dadurch gekennzeichnet, dass** sie, bezogen auf das Gewicht, 30 bis 50% Reiskleieöl, 15 bis 25% Sesamöl, 20 bis 40% Maisöl und 5 bis 15% Weizenkeimöl enthält.

3. Verfahren zur Herstellung einer Lipidzusammensetzung nach Anspruch 1, bei dem man Rohöle oder teilweise raffinierte Öle verwendet, **dadurch gekennzeichnet, dass** die Ölmischung von den Gummen befreit wird, entfärbt und desodoriert wird, und zwar bei Bedingungen, die die Aufrechterhaltung eines Gehalts an Unverseifbarem von mindestens 2 Gew.-% sowie einer Stabilität gegen Oxidation, die einer Induktionszeit von mindestens 15 h im Rancimattest bei 100°C enspricht, gestatten.

4. Verfahren nach Anspruch 3, **dadurch gekennzeichnet, dass** die Entfernung der Gummen stattfindet, indem die Ölmischung in Gegenwart von Wasser mit etwa 80°C mit einer konzentrierten Zitronensäurelösung in Kontakt gebracht wird, wobei man eine Hydratisierung der Gummen und dann eine Abtrennung der Gummen durch Zentrifugieren oder Dekantieren vornimmt.

5. Verfahren nach Anspruch 3, **dadurch gekennzeichnet, dass** die Entfernung der Gummen stattfindet, indem die durch Dampfzirkulation auf etwa 80°C erhitzte Ölmischung bei Unterdruck behandelt wird.

6. Verfahren nach Anspruch 3, **dadurch gekennzeichnet, dass** man nach Abtrennung der Gummen die von Gummen befreite Ölmischung behandelt, indem sie während etwa 20 min bei etwa 80-85°C bei einem Unterdruck von etwa 50-80 mbar mit einem aus feuchtem amorphem Siliciumoxidgel bestehenden Adsorptionsmittel in Kontakt gebracht wird.

7. Verfahren nach Anspruch 3, **dadurch gekennzeichnet, dass** man auch eine Bleichung mit Hilfe von mit Säure aktivierter Bleicherde vornimmt.

8. Verfahren nach Anspruch 3, **dadurch gekennzeichnet, dass** man die Ölmischung unter schonenden Bedingungen während etwa 2 h bei etwa 180°C mit etwa 1% Frischdampf unter einem Unterdruck von 1-2 mbar desodoriert.

9. Kosmetische Zusammensetzung, deren Lipidteil aus einer Lipidzusammensetzung nach Anspruch 1 oder 2 besteht.

10. Kosmetische Zusammensetzung nach Anspruch 9, **dadurch gekennzeichnet, dass** sie in wässriger Form als Lösung, Suspension oder Aerosol, Wasser-in-Öl-Emulsion, insbesondere Creme, oder Öl-in-Wasser-Emulsion, insbesondere Milch, oder in wasserfreier Form, insbesondere als Balsam, Körperöl, Sonnenschutzöl oder Lippenstift, vorliegt und **dass** sie ferner mindestens einen kosmetischen Zusatz enthält, der aus der aus folgenden Substanzen bestehenden Gruppe ausgewählt ist: Emulgatoren, Schweißschutzmittel, Stabilisatoren, Konservierungsmittel, Antioxidanzien, Sonnenfilter, Parfums, Farbstoffe, Weichmacher, Perlglanzmittel, Wachse und organische oder mineralische Füllstoffe.

11. Ernährungsergänzung für kosmetische Zwecke, deren Lipidteil aus einer Lipidzusammensetzung nach Anspruch 1 oder 2 besteht.

## Claims

1. Lipidic composition for cosmetic use, having an anti-ageing action, **characterized in that** it contains rice bran oil and sesame oil and 2 to 3 % by weight of unsaponifiables, and **in that** the fatty acids of the triglycerides comprise, by weight, 30 % to 40 % oleic acid, 40 to 50 % linoleic acid and less than 2 % alpha-linolenic acid.

2. Lipidic composition according to claim 1, **characterized in that** in contains, by weight, 30 to 50 % rice bran oil, 15 to 25 % sesame oil, 20 to 40 % maize oil and 5 to 15 % wheat germ oil.

3. Process for preparing a lipidic composition according to claim 1, wherein raw or partially refined oils are used, **characterized in that** the mixture of oils is degummed, decolorized and deodorized under conditions making it possible to maintain a concentration of unsaponifiables of at least 2 % by weight as well as a stability against oxidation corresponding to a period of induction of at least 15 h in the Rancimat test at 100°C.

4. Process according to claim 3, **characterized in that** degumming is carried out by putting the mixture of oils in contact with a concentrated solution of citric acid, in the presence of water at approximately 80°C during which hydration of the gums occurs, and this is then followed by separation of the gums, by centrifuging or decanting.

5. Process according to claim 3, **characterized in that** degumming is carried out by treating the mixture of oils by heating at approximately 80°C with steam circulation under vacuum.

6. Process according to claim 3, **characterized in that**, after separating the gums, the mixture of degummed oils is treated by putting it into contact with an adsorbent consisting of moist amorphous silica gel for approximately 20 min at approximately 80-85°C under a vacuum of approximately 50-80 mbar.

7. Process according to claim 3, **characterized in that** in addition bleaching is carried out with decolorizing earth activated with acid.

8. Process according to claim 3, **characterized in that** the mixture of oils is deodorized under controlled conditions, at approximately 180°C with approximately 1 % live steam and under a vacuum of approximately 1-2 mbar for approximately 2 h.

9. Cosmetic composition of which the lipidic part consists of a lipidic composition according to claim 1 or 2.

10. Cosmetic composition according to claim 9, **characterized in that** it is in aqueous form, as a solution, a suspension or aerosol, a water-in-oil emulsion, in particular a cream, or an oil-in-water emulsion, in particular a milk, or in anhydrous form, in particular as a balm, a body oil, an anti-sun oil or a lipstick, and **in that** it also contains at least one cosmetic additive chosen from the group formed of emulsifiers, anti-perspirant agents, stabilizers, preservatives, anti-oxidants, sun filters, perfumes, colorants, emollients, pearl agents, waxes and inorganic or organic fillers.

11. Nutritional supplement for a cosmetic purpose, of which the lipidic part consists of a lipidic composition according to claim 1 or 2.
